# EUROPEAN PATENT APPLICATION

(11) **EP 3 424 494 A1**
(43) Date of publication of application: **09.01.2019**
(21) Application number: 17180383.6
(22) Date of filing: 07.07.2017
(51) Int. Cl.: A61K 9/107, A61K 47/10, A61K 47/22, A61K 31/05, A61K 31/352

(54) **STABLE CANNABINOID COMPOSITIONS**

(71) Applicant: SolMic Research GmbH, 40225 Düsseldorf (DE)
(72) Inventor: KNÖLLER, Ilse, 41470 Neuss (DE); SOWIK, Thomas, 44653 Herne (DE)
(74) Representative: Schmitz, Joseph

(57) **Abstract**

A composition comprising a cannabinoid, in particular a phytocannabinoid or a synthetic cannabinoid, where the cannabinoid is stabilised against oxidation and/or photochemical degradation, characterized in that the composition comprises a micellar solution of poloxamer-based micelles in an aqueous solution and where the poloxamer micelles encapsulate the cannabinoid.

## Description

### TECHNICAL FIELD

The present invention relates to compositions comprising a lipophilic bioactive compounds such as a cannabinoid in which the cannabinoid is stabilized against oxidation and/or photochemical degradation.

### PRIOR ART

Pharmaceutical formulations allow active ingredients to enter the body and arrive at the targeted tissue so that they may develop the intended effect there. When an active ingredient has to be administered via a given route, much consideration should be given to the physico-chemical properties of the active ingredient and its formulation.

For instance, the uptake of an active ingredient by the intestinal tract via an oral route, i.e. the ability to enter the systemic circulation, is heavily dependent of its stability towards acids (gastric juice), enzymatic degradation (e.g. pancreatic enzymes) and water solubility.

Also, the uptake via the skin depends on the ability of the active ingredient to bypass the upper skin layer consisting of the upper epidermis with its skin cells and fibers without blood and lymph vessels, the lower dermis and into the subcutaneous environment with its blood vessels.

In the intestinal tract, lipophilic compounds are made water-soluble by the help of the bile salts, which have amphiphilic properties. With the help of the bile salts the lipophilic components are caged in small round shaped structures, so-called micelles, which consist of an inner lipophilic and outer water-soluble moiety and thus can render lipophilic ingredients, which are a priori water insoluble, water-soluble.

However, the endogenous processes have a relatively low efficacy. They need most often stimulation of the release of the bile salts from the gall bladder by the lipohilic compound itself and stimulation by concomitant food intake. The efficacy depends on the regular motility of the intestinal tract to bring the ingredients into contact with the bile salts to form an emulsion, as well as the amount of bile salts that can be released.

Usually, this process increases the uptake of lipophilic compounds up to 5-15% bioavailability, which is sufficient for supply in healthy, young adults. In diseased or elderly persons the regular processes may, however, not be sufficient to exert the desired effect of an active ingredient or maintain health and wellness, e.g. due to hypovitaminoses of vitamin A, D, E, and K and other essential factors such as ubiquinol/ubiquinone, or essential fatty acids. Moreover, natural extracts from fruits or vegetables which often have lipophilic or resin-like, instable constituents may not be taken up anymore.

Many processes to emulsify these ingredients are described in the literature and have been used for many years. However, these emulsions may have also low efficacy due to their instability in the intestinal tract, in which the emulsion may "break" due to dilution below critical micelle concentration and thereby rendering the micellar formulations instable.

The above-mentioned problems are further exacerbated in liquid or gel-like formulations which in most cases display insufficient shelf life, since degradation of the ingredient to be emulsified in response to light and oxygen exposure further reduces the availability of ingredients in an emulsion, when compared to solid forms such as lozenges or capsules.

Cannabinoids exhibit low solubility and stability in aqueous solutions and are therefore often formulated as oily solutions or dissolved in organic solvents which are rather unsuitable for ingestion or topical application. When formulated as oily solutions or dissolved in organic solvents the cannabinoids suffer from both oxidative and photochemical degradation, which quickly becomes analytically detectable.

Therefore, there is a need to provide formulations of otherwise instable, lipophilic active ingredients which exhibit improves stability of the ingredient when passing through the gastro-intestinal tract and which moreover display excellent shelf life, especially when exposed to irradiation, temperature variations and or sunlight.

### SUMMARY OF THE INVENTION

The compositions, as well as the process for obtaining such compositions, according to the present invention, provide for a means to formulate active ingredients, in particular lipophilic or resinous active ingredients such as cannabinoids, in way that they are readily available throughout the entire gastro-intestinal tract with high efficiency and moreover display enhanced stability against light and/or thermal degradation when compared with compositions according to the state of the art by encapsulating the cannabinoid in composite micelles of poloxamer and tocopherol-like compounds. This allows to provide formulations that can easily be stored under ambient conditions without special precautions which would otherwise be needed for compositions according to the state of the art. In particular, the formulations do not need to be refrigerated and/or kept in the dark.

In a first aspect, the present invention thus provides a composition comprising a lipophilic bioactive compound such as a cannabinoid, in particular a phytocannabinoid or a synthetic cannabinoid, where the cannabinoid is stabilised against oxidation and/or photochemical degradation, characterized in that the composition comprises a micellar solution of poloxamer and non-aqueous non-alkoxylated solvent micelles in an aqueous solution, and where the poloxamer and non-aqueous non-alkoxylated solvent micelles encapsulate the lipophilic bioactive compound and where the non-aqueous non-alkoxylated solvent has a formula: 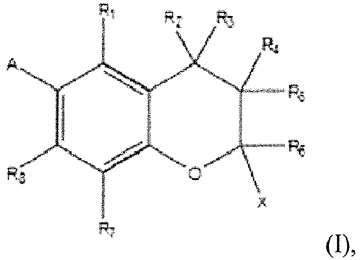 where A corresponds to H, SH, NH₂, COOH, CONH₂ or OH or a C1-C8 alkyl segment or C2-C8 alkenyl segment at least bearing one H, SH, NH₂, COOH, CONH₂ or OH, X corresponds to a linear or branched alkyl or alkenyl chain, and R₁, R₂, R₃, R₄, R₅, R₇, R₈ independently of each other correspond to either H or CH₃; and preferably where A corresponds to OH, where X corresponds to a linear or branched alkyl or alkenyl chain, and R₁, R₂, R₃, R₄, R₅, R₇, R₈ independently of each other correspond to either H or CH₃.

In a second aspect the present invention thus provides a process for stabilizing a lipophilic bioactive compound such as a cannabinoid, in particular a phytocannabinoid or a synthetic cannabinoid, against oxidation and/or photochemical degradation, comprising the steps of, in this order:
a. heating an amount of a poloxamer to a first temperature such as to form a melt of the poloxamer,
b. adding an amount of a non-aqueous non-alkoxylated solvent having a formula 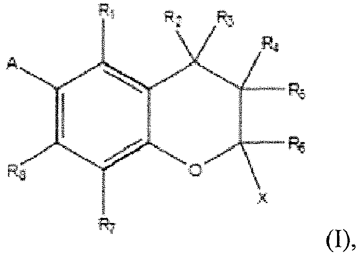 where A corresponds to H, SH, NH₂, COOH, CONH₂ or OH or a C1-C8 alkyl segment or C2-C8 alkenyl segment at least bearing one H, SH, NH₂, COOH, CONH₂ or OH, where X corresponds to a linear or branched alkyl or alkenyl chain, and R₁, R₂, R₃, R₄, R₅, R₇, R₈ independently of each other correspond to either H or CH₃; and preferably where A corresponds to OH, where X corresponds to a linear or branched alkyl or alkenyl chain, and where R₁, R₂, R₃, R₄, R₅, R₇, R₈ independently of each other correspond to either H or CH₃ to the melt of the poloxamer and mixing such as to dissolve the non-aqueous non-alkoxylated solvent in the melt of the poloxamer and thereby forming a first homogenous liquid mixture, while maintaining the temperature of the first homogenous liquid mixture within 10 °C of the first temperature with the proviso that the temperature corresponds at least to the melting temperature of poloxamer,
c. adding an amount of a cannabinoid to the first homogenous liquid mixture and mixing such as to dissolve the cannabinoid in the first homogenous liquid mixture and thereby forming a second homogenous liquid mixture, while maintaining the temperature of the second homogenous liquid mixture within 10 °C of the first temperature with the proviso that the temperature corresponds at least to the melting temperature of poloxamer,
d. adding an amount of an aqueous solution of a carboxylic acid having two or more carboxyl moieties to the second homogenous liquid mixture, wherein the temperature of the aqueous solution of a carboxylic acid having two or more carboxyl moieties is within 10° C of the first temperature with the proviso that the temperature corresponds at least to the melting temperature of poloxamer, and mixing such as to form a micellar solution of poloxamer and non-aqueous non-alkoxylated solvent micelles encapsulating the cannabinoid in the aqueous solution of a carboxylic acid having two or more carboxyl moieties,
e. reducing the temperature of the micellar solution to a temperature below the melting temperature of poloxamer and non-aqueous non-alkoxylated solvent.

In a third aspect, the present invention thus provides a pharmaceutical formulation comprising a composition according to the first aspect of the present invention.

In a fourth aspect, the present invention thus provides a use of a composition according to the first aspect of the present invention for reducing oxidation and/or photochemical degradation of a lipophilic bioactive compound such as a cannbinoid.

In a fifth aspect, the present invention thus provides a use of a composition according to the first aspect of the present invention in a pharmaceutical formulation.

Further embodiments of the invention are laid down in the dependent claims.

### BRIEF DESCRIPTION OF THE DRAWINGS

Preferred embodiments of the invention are described in the following with reference to the drawings, which are for the purpose of illustrating the present preferred embodiments of the invention and not for the purpose of limiting the same. In the drawings,

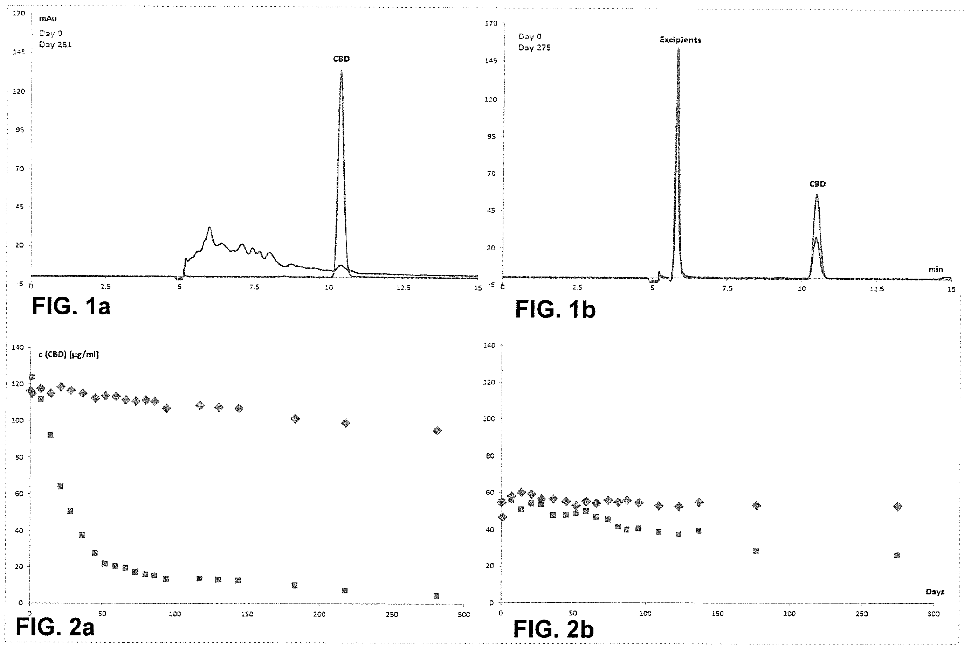

- Fig. 1a: shows two chromatograms of a solution of cannabidiol (CBD), whereas one chromatogram was measured on the day of preparation of the solution (day 0, continuous line) and the other was measured 275 days after preparation (dotted line), during which the solution was kept at room temperature and was exposed to natural light.
- Fig. 1b: shows two chromatograms of a stabilized composition of cannabidiol (CBD) according to the present invention, whereas one chromatogram was measured on the day of preparation of the composition (day 0, continuous line) and the other was measured 275 days after preparation (dotted line), during which the composition was kept at room temperature and was exposed to natural light.
- Fig. 2a: shows two time-dependent measurement series of cannabidiol (CBD) content in µg/ml, in a solution of cannabidiol (CBD) which was shielded from exposure to light (lozenges) and in a solution of cannabidiol (CBD) that was exposed to light (squares).
- Fig. 2b: shows two time-dependent measurement series of cannabidiol (CBD) content in µg/ml, in a stabilized composition of cannabidiol (CBD) according to the present invention which was shielded from exposure to light (lozenges) and in a solution of cannabidiol (CBD) that was exposed to light (squares).

### DESCRIPTION OF PREFERRED EMBODIMENTS

It is understood that the compounds used in the composition, in particular the lipophilic bioactive compound, poloxamers and non-aqueous non-alkoxylated solvent and others may be of pharmaceutically acceptable grade, i.e. pharmaceutically acceptable.

It is understood that the process according to the present invention, during steps a. to d. or e., the formed melts and mixtures liquids or gels are processed by agitating the melts, liquids or gels such as to ensure thorough mixing and formation of homogenous solutions or mixtures.

In a first aspect, the present invention thus provides a stabilized composition comprising a lipophilic bioactive compound such as a cannabinoid, in particular a phytocannabinoid or a synthetic cannabinoid, where the lipophilic bioactive compound such as a cannabinoid is stabilised against oxidation and/or photochemical degradation, characterized in that the composition comprises a micellar solution of poloxamer and non-aqueous non-alkoxylated solvent micelles in an aqueous solution, and where the poloxamer and non-aqueous non-alkoxylated solvent micelles encapsulate the lipophilic bioactive compound such as a cannabinoid and where the non-aqueous non-alkoxylated solvent has a formula: 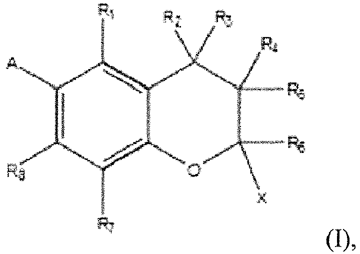 where A corresponds to H, SH, NH₂, COOH, CONH₂ or OH or a C1-C8 alkyl segment or C2-C8 alkenyl segment at least bearing one H, SH, NH₂, COOH, CONH₂ or OH, X corresponds to a linear or branched alkyl or alkenyl chain, and R₁, R₂, R₃, R₄, R₅, R₇, R₈ independently of each other correspond to either H or CH₃; and preferably where A corresponds to OH, where X corresponds to a linear or branched alkyl or alkenyl chain, and R₁, R₂, R₃, R₄, R₅, R₇, R₈ independently of each other correspond to either H or CH₃.

In a preferred embodiment, the composition according to the present invention is in the form of a liquid or a gel comprising a micellar solution of poloxamer and non-aqueous non-alkoxylated solvent micelles in an aqueous solution, and where the poloxamer and non-aqueous non-alkoxylated solvent micelles encapsulate the lipophilic bioactive compound such as a cannabinoid. Encapsulating in micelles the lipophilic bioactive compound helps on one hand to solubilize the lipophilic bioactive compound in aqueous environments and on the other hand helps stabilizing the lipophilic bioactive compound such as a cannabinoid against degradation, and no further organic solvents are needed to increase the content of the lipophilic bioactive compound such as a cannabinoid.

In a preferred embodiment, in the composition according to the present invention, the lipophilic bioactive compound may be chosen from lipophilic bioactive compounds that are isolated from plants or animals and in particular such lipophilic bioactive compounds which are light-sensitive. This includes compounds such as lipophilic vitamins such as vitamin A, flavonoids, cannabinoids, ubiquinol/ubiquinone, phytosterols, phytoestrogens, polyphenols, anthocyanins, omega-3 fatty acids, carotenoids such as lutein, astaxanthine, beta-carotene. Alternatively, the lipophilic bioactive compound may be an active pharmaceutical ingredient. In particular, the lipophilic bioactive compound may be chosen among cannabinoids such as cannabidiol or tetrahydrocannabinol. Cannabidiol (CBD) is one of at least 113 active cannabinoids identified in cannabis. It is a major phytocannabinoid, accounting for up to 40% of the plant's extract and has been considered to have a wide scope of potential medical applications - due to clinical reports showing the lack of side effects, particularly a lack of addictive potential. It is notorious for being particularly instable when exposed to light and/or heat and is therefore stored as crystalline solid since solutions cannot be stored for more than a few days. Cannabidiol is furthermore sparingly soluble in aqueous solutions, which is why solubility and hence, bioavailability, can be increased by providing a micellar solution of micelles encapsulating the cannabidiol in an aqueous solution. The same can be said of tetrahydrocannabinol.

In a preferred embodiment, in the composition according to the present invention, the non-aqueous non-alkoxylated solvent is a tocopherol such as alpha-, beta-, gamma- or delta-tocopherol or tocotrienol such as alpha-, beta-, gamma- or delta-tocotrienol. Tocopherol and tocotrienol are readily available commercially from sources such vegetable oils, nuts and seeds. While they help the formation of micelles that are particularly stable, furthermore some of the tocopherols and/or tocotrienols useful in the present invention also have vitamin E activity. Without wishing to be bound to a particular theory, it is believed that the use of tocopherols, which are structurally similar to cannabinoids in particular, in conjunction with one or more poloxamers provides for the stabilization effect observed in the compositions according to the present invention.

In a preferred embodiment, in the composition according to the present invention, the lipophilic bioactive compound such as a cannabinoid is present in an amount of 0.1 - 10 weight percent, preferably of 0.1 to 7 weight respect, with respect to the total weight of the composition.

In a preferred embodiment, in the composition according to the present invention, the lipophilic bioactive compound such as a cannabinoid is present in an amount of 0.1 - 10 weight percent, preferably of 0.1 to 7 weight percent, with respect to the total weight of the composition, and the poloxamer and non-aqueous non-alkoxylated solvent may be present in an amount of 10 to 80 weight percent, preferably of 15 to 25 weight percent with respect to the total weight of the composition, whereas the remainder is formed by an aqueous solution, preferably an aqueous solution of a carboxylic acid having two or more carboxyl moieties.

While the composition according to the present invention are able to accommodate lipophilic bioactive compounds in an amount of 0.1 - 10 weight percent, with respect to the total weight of the composition, the amount of cannabinoids is usually in the range of of 0.1 - 3 weight percent, the amount of krill oil and/or astaxanthin is usually in the range of 0.1 - 6.5 weight percent, the amount of graviola plant extract is usually in the range of of 0.1 - 8 weight percent, the amount of curcuma plant extract is usually in the range of of 0.5 - 4 weight percent, the amount of ubichinone is usually in the range of 0.1 - 5 weight percent and the amount of propolis and Siberian ginseng plant extract is usually in the range of 0.1 - 4 weight percent.

In a preferred embodiment, in the composition according to the present invention, the aqueous solution may be formed by an aqueous solution of a carboxylic acid having two or more carboxyl moieties or a salt thereof. In particular, the aqueous solution of a carboxylic acid having two or more carboxyl moieties or a salt thereof may further comprise one or more carboxylic acids having one carboxyl moiety or a salt thereof. Examples of a suitable carboxylic acid having two or more carboxyl moieties or a salt thereof are acids such as tricarboxylic acids like citric acid or its mono-, di- or tri-salts.

In a preferred embodiment, in the composition according to the present invention, the composition may comprise a mixture of poloxamers, such as for example a mixture of a poloxamer having a higher molecular weight and a poloxamer having a lower molecular weight, wherein the poloxamer having a higher molecular weight is present in a weight ratio of 2:1 to 1:1 such as for example approximately 4:1, with respect to the non-aqueous non-alkoxylated solvent.

In a preferred embodiment, in the composition according to the present invention, the weight ratio between the poloxamer and the non-aqueous non-alkoxylated solvent is of from 9:1 to 199:1 and preferably is from 9:1 to 39:1. This ratio allows formation of stable composite micelles of poloxamer and non-aqueous non-alkoxylated solvent that further allow for good bioavailability of the lipophilic bioactive compound such as a cannabinoid. For example, in the case where the non-aqueous non-alkoxylated solvent is a tocopherol, the weight ratio between the poloxamer and the tocopherol can be within the above range such as for example approximately 9:1, 14:1, 19:1.

In a second aspect, the present invention provides a process for stabilising a lipophilic bioactive compound such as a cannabinoid, in particular a phytocannabinoid or a synthetic cannabinoid, against oxidation and/or photochemical degradation, comprising the steps of, in this order:
a. heating an amount of a poloxamer to a first temperature such as to form a melt of the poloxamer,
b. adding an amount of a non-aqueous non-alkoxylated solvent having a formula 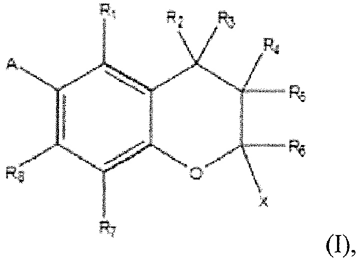 where A corresponds to H, SH, NH₂, COOH, CONH₂ or OH or a C1-C8 alkyl segment or C2-C8 alkenyl segment at least bearing one H, SH, NH₂, COOH, CONH₂ or OH, X corresponds to a linear or branched alkyl or alkenyl chain, and R₁, R₂, R₃, R₄, R₅, R₇, R₈ independently of each other correspond to either H or CH₃; and more preferably where A corresponds to OH, where X corresponds to a linear or branched alkyl or alkenyl chain, and R₁, R₂, R₃, R₄, R₅, R₇, R₈ independently of each other correspond to either H or CH₃ and more preferably where A corresponds to OH, where X corresponds to a linear or branched alkyl or alkenyl chain, and R₁, R₂, R₃, R₄, R₅, R₇, R₈ independently of each other correspond to either H or CH₃, to the melt of the poloxamer and mixing such as to dissolve the non-aqueous non-alkoxylated solvent in the melt of the poloxamer and thereby forming a first homogenous liquid mixture, while maintaining a first mixing temperature of the first homogenous liquid mixture within 10 °C of the first temperature with the proviso that the first mixing temperature corresponds at least to the melting temperature of poloxamer,
c. adding an amount of lipophilic bioactive compound such as a cannabinoid to the first homogenous liquid mixture and mixing such as to dissolve the lipophilic bioactive compound such as a cannabinoid in the first homogenous liquid mixture and thereby forming a second homogenous liquid mixture, while maintaining a second mixing temperature of the second homogenous liquid mixture within 10 °C of the first temperature with the proviso that the second mixing temperature corresponds at least to the melting temperature of poloxamer,
d. adding an amount of an aqueous solution of a carboxylic acid having two or more carboxyl moieties to the second homogenous liquid mixture, wherein a third mixing temperature of the aqueous solution of a carboxylic acid having two or more carboxyl moieties is within 10° C of the first temperature with the proviso that the third mixing temperature corresponds at least to the melting temperature of poloxamer, and mixing such as to form a micellar solution of poloxamer and non-aqueous non-alkoxylated solvent micelles encapsulating the cannabinoid in the aqueous solution of a carboxylic acid having two or more carboxyl moieties,
e. reducing the temperature of the micellar solution to a temperature below the melting temperature of poloxamer and non-aqueous non-alkoxylated solvent.

The lipophilic bioactive compounds can thus be protected against oxidation and/or photochemical degradation by stabilizing them in the form of a micellar solution of poloxamer and non-aqueous non-alkoxylated solvent micelles encapsulating the lipophilic bioactive compounds in an aqueous solution. The thus stabilized lipophilic bioactive compounds exhibit good shelf life at room temperature and further eliminates the need for including a protective gas in the container in which the stabilized lipophilic bioactive compounds are stored. The quality of the micellar formation can, in most cases, be verified by visual inspection. When the composition appears optically transparent (in opposition to turbid), the formation of appropriate micelles has likely occurred. The above non-withstanding, the micelles formed according to the present invention should exhibit a mean diameter of less than 100 nm, and preferably have a mean diameter of between 10 nm and 60 nm when measured with dynamic light scattering (DLS).

The process for stabilising a lipophilic bioactive compound according to the present invention may be carried out in suitable vessels equipped with temperature-control means as well as mixing means, such as for example a magnetic stirrer.

In a preferred embodiment of the process for stabilising a lipophilic bioactive compound, the step e. of reducing the temperature of the micellar solution to a temperature below the melting temperature of poloxamer and non-aqueous non-alkoxylated solvent the may be carried out concomitantly with step d., and in particular after the addition of the aqueous solution of a carboxylic acid and concomitantly with the ensuing mixing sub-step.

In a third aspect, the present invention also provides a pharmaceutical formulation comprising a composition according to the first aspect of the present invention. The pharmaceutical formulation may be chosen mainly, but not exclusively, from oral or topical formulations.

In particular, when the composition is provided in an oral formulation, the composition may be used as-is or may be combined with other excipients such as for example flavoring agents, antioxidants and so on. The oral formulation may be a liquid or sirup, gel or encapsulated gel. When the composition is provided in a topical formulation, the composition may be used as-is or may be combined with other excipients such as for example oil or in general emollients, stabilizers and so on. The topical formulation may be a cream, gel, liniment or balm, lotion, or ointment, etc.. In an alternative embodiment, the topical formulation comprising the composition is a transdermal patch of a lipophilic bioactive compound such as a cannabinoid, which preferably continuously releases the lipophilic bioactive compound such as a cannabinoid through the skin and into the bloodstream, i.e. is a transdermal patch providing extended release of a lipophilic bioactive compound such as a cannabinoid.

In a fourth aspect, the present invention thus provides a use of a composition according to the first aspect of the present invention for reducing oxidation and/or photochemical degradation of a lipophilic bioactive compound such as a cannabinoid. The composition can thus be used for providing lipophilic bioactive compounds such as a cannabinoid where the lipophilic bioactive compound such as a cannabinoid is stabilized in a micelle formed from poloxamer and tocopherol and in which the compound is protected from the influence of radiation, especially natural light and photooxidation.

In a fifth aspect, the present invention thus provides a use of a composition according to the first aspect of the present invention in a pharmaceutical formulation such as for example a topical oral formulation.

Further embodiments of the invention are laid down in the dependent claims.

### EXAMPLES

4 parts by weight of a first poloxamer (commercially obtainable from BASF Germany under the trademark Kolliphor® P 188) were combined with 16 parts by weight of a second poloxamer (commercially obtainable from BASF Germany under the trademark Kolliphor® P 407) in a beaker and heated to a temperature of between 70 and 80 °C until a first colorless and transparent melt was formed. Subsequently, 1 part by weight of a DL-alpha-tocopherol (commercially obtainable from BASF Germany) was added to the first melt while maintaining a temperature of between 70 and 80 °C until a second yellowish and transparent melt was formed. Subsequently, 1 part by weight of cannabidiol as natural extract was added to the second melt while maintaining a temperature of between 70 and 80 °C until the cannabidiol was dissolved, thereby forming a third transparent melt. Then, 88 parts by weight of an aqueous solution containing 0.1 parts by weight of potassium sorbate and 0.05 parts by weight of citric acid was preheated to a temperature of between 70 and 80 °C was added to the third melt under agitation such as to disperse the water within the third melt and form a pale yellow and transparent which was then cooled to 4°C to form a stabilized composition comprising cannabidiol.

As a comparative composition of cannabidiol, a solution of cannabidiol comprising 1 w/w-% cannabidiol in acetonitrile:water (1:1) was used.

Both compositions were then analyzed by RP-HPLC on day 0 and again after 275 days of exposure to natural light at room temperature. As can be seen in Figure 1a, the area under the peak corresponding to cannadibiol (CBD) almost disappeared in the solution of cannabidiol after 275 days (dotted line) whereas as can be seen in Fig 1b, in the stabilized composition, the area under the peak corresponding to cannabidiol (CBD) was reduced by approximately 45% (dotted line). Thus, this indicates that when compared to solutions of organic solvents, stabilized compositions of cannabidiol have a decreased tendency to degrade the cannabidiol.

Furthermore, both the solution of cannabidiol comprising 1 w/w-% cannabidiol in acetonitrile:water (1:1) and the stabilized composition were placed in a light-protected vessel and exposed to room temperature for 275 days in a further experiment in which the concentration of cannabidiol was measured. As can be seen from Figure 2a, the absence of light exposure resulted in a significant decrease in degradation of cannabidiol in the sample of solution of cannabidiol comprising 1 w/w-% cannabidiol (lozenges), when compared to the case where an identical sample was exposed to light (squares). In contrast, as can be seen from Figure 2b, the difference in degradation of cannabidiol was less marked in the case of the stabilized composition where the unexposed sample (lozenges) and exposed samples (squares) varied less, further indicating that photochemical degradation and/or photooxidation is at the root of the degradation of cannabidiol in the analyzed samples and that stabilization through micellization reduces photochemical degradation of cannabidiol.

## Claims

1. A composition comprising a lipophilic bioactive compound such as a cannabinoid, in particular a phytocannabinoid or a synthetic cannabinoid, where the lipophilic bioactive compound is stabilised against oxidation and/or photochemical degradation, **characterized in that** the composition comprises a micellar solution of poloxamer and non-aqueous non-alkoxylated solvent micelles in an aqueous solution, and where the poloxamer and non-aqueous non-alkoxylated solvent micelles encapsulate the lipophilic bioactive compound and where the non-aqueous non-alkoxylated solvent has a formula: 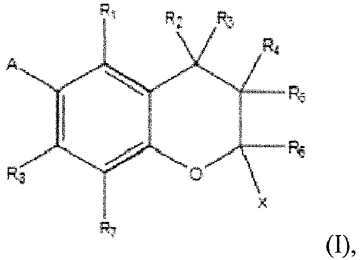 where A corresponds to H, SH, NH₂, COOH, CONH₂ or OH or a C1-C8 alkyl segment or C2-C8 alkenyl segment at least bearing one H, SH, NH₂, COOH, CONH₂ or OH, X corresponds to a linear or branched alkyl or alkenyl chain, and R₁, R₂, R₃, R₄, R₅, R₇, R₈ independently of each other correspond to either H or CH₃; and more preferably where A corresponds to OH, where X corresponds to a linear or branched alkyl or alkenyl chain, and R₁, R₂, R₃, R₄, R₅, R₇, R₈ independently of each other correspond to either H or CH₃.

2. The composition according to claim 1, wherein the cannabinoid is either cannabidiol or tetrahydrocannabinol.

3. The composition according to claim 1 or 2, wherein the non-aqueous non-alkoxylated solvent is a tocopherol such as alpha-, beta-, gamma- or delta-tocopherol or tocotrienol such as alpha-, beta-, gamma- or delta-tocotrienol.

4. The composition according to any of the preceding claims, wherein the weight ratio between the poloxamer and the non-aqueous non-alkoxylated solvent is of from 9:1 to 99:1 and preferably is from 9:1 to 39:1 and wherein the composition further comprises a glycerol in an amount of up to 10 weight percent..

5. The composition according to any of the preceding claims, wherein the lipophilic bioactive compound such as a cannabinoid is present in an amount of 0.1 - 5 weight percent, preferably of 0.1 to 2 weight percent, with respect to the total weight of the formulation.

6. A pharmaceutical formulation comprising a composition according to any of the preceding claims, wherein the pharmaceutical formulation is an oral or topical pharmaceutical formulation.

7. A process for stabilising a lipophilic bioactive compound such as a cannabinoid, in particular a phytocannabinoid or a synthetic cannabinoid, against oxidation and/or photochemical degradation, comprising the steps of, in this order:
a. heating an amount of a poloxamer to a first temperature such as to form a melt of the poloxamer,
b. adding an amount of a non-aqueous non-alkoxylated solvent having a formula 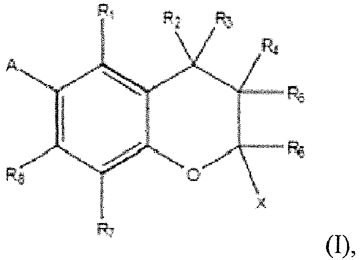 where A corresponds to H, SH, NH₂, COOH, CONH₂ or OH or a C1-C8 alkyl segment or C2-C8 alkenyl segment at least bearing one H, SH, NH₂, COOH, CONH₂ or OH, X corresponds to a linear or branched alkyl or alkenyl chain, and R₁, R₂, R₃, R₄, R₅, R₇, R₈ independently of each other correspond to either H or CH₃; and more preferably where A corresponds to OH, where X corresponds to a linear or branched alkyl or alkenyl chain, and R₁, R₂, R₃, R₄, R₅, R₇, R₈ independently of each other correspond to either H or CH₃ and more preferably where A corresponds to OH, where X corresponds to a linear or branched alkyl or alkenyl chain, and R₁, R₂, R₃, R₄, R₅, R₇, R₈ independently of each other correspond to either H or CH₃ to the melt of the poloxamer and mixing such as to dissolve the non-aqueous non-alkoxylated solvent in the melt of the poloxamer and thereby forming a first homogenous liquid mixture, while maintaining a first mixing temperature of the first homogenous liquid mixture within 10 °C of the first temperature with the proviso that the first mixing temperature corresponds at least to the melting temperature of poloxamer,
c. adding an amount of lipophilic bioactive compound such as a cannabinoid to the first homogenous liquid mixture and mixing such as to dissolve the lipophilic bioactive compound such as a cannabinoid in the first homogenous liquid mixture and thereby forming a second homogenous liquid mixture, while maintaining a second mixing temperature of the second homogenous liquid mixture within 10° C of the first temperature with the proviso that the second mixing temperature corresponds at least to the melting temperature of poloxamer,
d. adding an amount of an aqueous solution, preferably an aqueous solution of a carboxylic acid having two or more carboxyl moieties to the second homogenous liquid mixture, wherein a third mixing temperature of the aqueous solution of a carboxylic acid having two or more carboxyl moieties is within 10° C of the first temperature with the proviso that the third mixing temperature corresponds at least to the melting temperature of poloxamer, and mixing such as to form a micellar solution of poloxamer and non-aqueous non-alkoxylated solvent micelles encapsulating the cannabinoid in the aqueous solution of a carboxylic acid having two or more carboxyl moieties,
e. reducing the temperature of the micellar solution to a temperature below the melting temperature of poloxamer and non-aqueous non-alkoxylated solvent.

8. The process according to claim 7, wherein the non-aqueous non-alkoxylated solvent is a tocopherol such as alpha-, beta-, gamma- or delta-tocopherol or tocotrienol such as alpha-, beta-, gamma- or delta-tocotrienol and/or the poloxamer has a central hydrophobic chain of poly(propylene oxide) flanked by two hydrophilic chains of poly(ethylene oxide).

9. The process according to claim 7 or 8, wherein the weight ratio between the poloxamer and the non-aqueous non-alkoxylated solvent is of from 9:1 to 99:1 and preferably is from 9:1 to 39:1.

10. The process according to any of the claims 7 to 9, wherein after step a. and before step b., the process further comprises the step of optionally adding an amount of a pharmaceutically acceptable polyol, in particular glycerol, to the melt of the poloxamer, and mixing such as to dissolve the pharmaceutically acceptable polyol in the melt of the poloxamer, while maintaining the temperature of the second homogenous liquid mixture within 10° C of the first temperature with the proviso that the temperature corresponds at least to the melting temperature of poloxamer

11. The process according to any of claims 7 to 10, wherein the first temperature is between 50 and 99 °C, preferably between 60 and 90 °C.

12. Use of a composition according to any of the claims 1 to 5 for reducing oxidation and/or photochemical degradation of a lipophilic bioactive compound such as a cannabinoid.

13. Use of a composition according to any of claims 1 to 5 in a pharmaceutical formulation, preferably in an oral or topical pharmaceutical formulation.
